# EUROPEAN PATENT APPLICATION

(11) **EP 2 311 947 A1**
(43) Date of publication of application: **20.04.2011**
(21) Application number: 09172961.6
(22) Date of filing: 14.10.2009
(51) Int. Cl.: C12N 15/09, C12P 21/00

(54) **Protein synthesis via click chemistry**

(71) Applicant: Ludwig-Maximilians-Universität München, 80539 München (DE); Bayer Schering Pharma AG, 13353 Berlin (DE)
(72) Inventor: Carell, Thomas, 82152, Krailling (DE)
(74) Representative: Weiss, Wolfgang

(57) **Abstract**

The present invention refers to a method of incorporating non-genetically encoded amino acids at single or multiple positions of a protein and optionally the further modification of the side chains of said non-genetically encoded amino acids, e.g. by introduction of glycan groups. Further, the present invention refers to novel single or multiple glycosylated proteins.

## Description

The present invention refers to a method of incorporating non-genetically encoded amino acids at single or multiple positions of a protein and optionally the further modification of the side chains of said non-genetically encoded amino acids, e.g. by introduction of glycan groups. Further, the present invention refers to novel single or multiple glycosylated proteins.

Eukaryotic proteins and in particularly cell surface proteins are frequently glycosylated. Protein glycosylation is essential for the proper functioning of the respective proteins and in the case of glycosylated protein therapeutics such as Epo the activity of the protein strongly depends on proper glycosylation⁽¹⁾. The synthesis and production of glycosylated proteins to either elucidate their biochemical function or to bring a glycosylated protein as a new therapeutic to the market is a formidable challenge⁽²⁾. The current most widely used methods for the production of glycosylated proteins involve overexpression of the protein in particular host systems such as mammalian or plant cell cultures, moss cultures or tobacco plants, to name a few which are able to make either partial or even fully glycosylated proteins⁽³⁻⁵⁾. Alternatively, the solid phase synthesis of glycosylated peptides and their insertion into the protein at question via native or expressed chemical ligation has emerged as a powerful chemical strategy⁽⁶⁻¹⁰⁾. The synthesis of proper glycosylated proteins remains to be one of the greatest challenges in organic chemistry⁽²⁾.

A possible approach to produce glycosylated proteins would be to insert unnatural amino acids having reactive side chains into such proteins. These amino acids would enable site-specific protein modification, e.g. glycosylation, by coupling to suitable reaction partners. Incorporation of unnatural mostly aromatic amino acids into proteins can be efficiently achieved via the amber suppression technique with the help of a specially evolved, orthogonal tyrosyl-tRNA synthetase/tRNA_{CUA} pair⁽¹⁵⁾. Alternatively the amber suppression with different *Methanosarcina* MS pyrrolysyl tRNA synthetase/tRNA_{CUA} pair was reported^{(14, 16-18)}.

The incorporation of a glycosylated non-genetically encoded amino acid into proteins is described by the group of P.G. Schultz ⁽²⁷⁾. Meanwhile, this publication has been retracted because the authors were unable to reproduce the results⁽²⁸⁾.

Here we report the incorporation of an artificial alkyne or alkene amino acid at single or multiple positions into the YFP protein and the subsequent glycosylation of these sites with different sugar azides using the Huisgen-Meldal-Sharpless click chemistry method^{(12,13)} which was used in our group for the synthesis of multiple sugar modified oligonucleotides⁽¹⁴⁾.

Here we show that the system *Methanosarcina mazei* (*Mm*) MS pyrrolysyl tRNA synthetase/tRNA_{CUA} pair has the potential to read though one or more than just one amber stop codon present in the messenger RNA to achieve insertion of an artificial, e.g. alkyne- or alkene-modified amino acid at one or more positions.

Thus, the subject matter of the present invention is a method of incorporating a non-genetically encoded amino acid into a protein, comprising:
(a) providing a nucleic acid molecule encoding said protein, wherein said nucleic acid molecule encodes a protein and comprises an amber stop codon (UAG) at one or more positions within the region encoding the open reading frame of said protein where a genetically encoded amino acid is to be replaced by said non-genetically encoded acid,
(b) expressing said nucleic acid molecule in an expression system capable of amber codon suppression, in the presence of said non-genetically encoded amino acid under conditions wherein the non-genetically encoded amino acid is incorporated into said protein at said one or more positions, and (c) recovering the expression product.

In a preferred embodiment, the side chains of the non-genetically encoded amino acid comprise reactive groups, which are suitable for the coupling of functional molecules thereto, e.g. reporter molecules or glycan molecules. Thus, in a further step (d), functional molecules, e.g. glycan molecules may be coupled to all positions of the protein where a genetically encoded amino acid has been replaced by a non-genetically encoded amino acid.

A further aspect of the present invention refers to a single or multiple functionalized, e.g. glycosylated protein where functional, e.g. glycan groups are attached to the protein backbone at the side chain of a non-genetically encoded amino acid, particularly via N-heterocyclic linkage groups.

Still a further aspect of the present invention refers to a nucleic acid molecule encoding a protein, wherein said nucleic acid molecule comprises an amber stop codon (UAG) at one or more positions within the region encoding the open reading frame of said protein, where a genetically encoded amino acid is to be replaced by said non-genetically encoded acid.

The present invention involves the incorporation of a non-genetically encoded amino acid at one or more positions into a protein, wherein the protein is synthesized in an expression system, e.g. a host cell or a cell-free translation system. The expression system is capable of single or multiple amber codon (UAG) suppression, i.e. protein synthesis does not stop at the amber codon but continues by incorporating a non-genetically encoded amino acid.

Step (a) of the method of the invention comprises providing a nucleic acid molecule encoding a protein into which a non-genetically encoded amino acid is to be incorporated. For this purpose, the nucleic acid molecule, e.g. a DNA- or RNA-molecule comprises amber stop codons (UAG) at one or more positions within the region encoding the open reading frame where a genetically encoded amino acid is to be replaced by the non-genetically encoded amino acid. The introduction of one or more UAG triplets into the nucleic acid molecule may be carried out by standard methods, e.g. by site-directed mutagenesis, as known to the skilled person.

Step (b) of the method of the invention involves the expression of the nucleic acid molecule in a suitable expression system system capable of amber codon suppression. The expression system may be a host cell, e.g. a prokaryotic host cell, such as *E.coli* or an *in vitro* translation system, e.g. a prokaryotic *in vitro* translation system. Alternatively, the expression system may be a eukaryotic expression system., e.g. a mammalian, i.e. human cell culture, a yeast cell or a plant cell. It should be noted that the expression system of the present invention involves enzymatic translation of protein-coding nucleic acids.

The nucleic aicd molecule may be introduced into the expression system by standard methods, e.g. by standard transformation techniques of bacterial host cells. The expression of the nucleic acid molecule in a host cell may be provided by operative linkage to an expression control sequence, e.g. a promoter, for example a bacterial promoter. The nucleic acid molecule may be present on a recombinant vector, e.g. a plasmid vector, which comprises further genetic elements for propagation in a host cell, e.g. an origin of replication, and a selection marker.

The expression system is capable of amber codon suppression. For this purpose, the expression system preferably comprises an amber codon suppressing tRNA synthetase/tRNA_{CUA} pair. Preferably, the expression system comprises a pyrrolysyl tRNA synthetase/tRNA_{CUA} pair, particularly from *Methanosarcina,* more particularly from *Methanosarcina mazei.* In a preferred embodiment, nucleic acid molecules encoding for the tRNA synthetase and the tRNA_{CUA} are provided on suitable expression vectors and introduced into a heterologous host cell, e.g. prokaryotic host cell such as *E.coli* or a eukaryotic cell.

The expression in step (b) of the method of the invention is carried out in the presence of sufficient amounts of the non-genetically encoded amino acid, which allow sufficient incorporation into the protein at positions of the amber stop codons. If the expression takes place in a host cell, the non-genetically encoded amino acid may be added to the culture medium in an amount of e.g. 10 µM to 100 mM, preferably 100 µM to 10 mM. Alternatively, if an *in vitro* translation system is used, corresponding amounts of the non-genetically encoded amino acid may be added to the *in vitro* translation system.

The non-genetically encoded amino acid which is incorporated into the protein of interest preferably comprises a reactive group suitable for the coupling of functional molecules, more preferably, the reactive unsaturated group, e.g. an alkyne or alkene group.

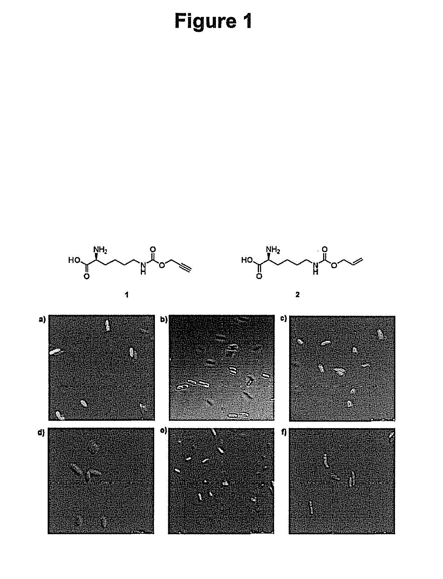

The non-genetically encoded amino acid is preferably a modified lysine derivative, wherein the ε-amino group of the lysine side chain is functionalized e.g. as shown in the following: 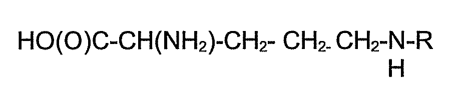 wherein R is a substituent containing a reactive group, e.g. an alkyne, alkene, diene, heterodiene, hydrazine or hydroxylamino containing substituent, which may have up to 20, e.g. up to 12 or up to 6 carbon atoms. Specific examples of modified lysine derivatives are shown in Fig. 1 (Compounds 1 and 2).

In the method of the invention, a non-genetically encoded amino acid is incorporated at one or more positions into a protein. The protein may be any protein, e.g. a eukaryotic, particularly mammalian including human protein, a prokaryotic protein or a genetically engineered, non-naturally occurring protein, e.g. an antibody, antibody fragment or a single chain antibody. In a preferred embodiment, one or more naturally occurring lysine residues are replaced by non-genetically encoded amino acid residues. The number of amino acid replacement positions may be 1, 2, 3, 4, or even more, e.g. up to 6 or 8. Specific examples of modified proteins are Yellow Fluorescence Proteins YFP mut 1 comprising 1 alkyne-containing amino acid (SEQ ID NO: 1) or YFP mut 3 comprising 3 alkyne-containing amino acids (SEQ ID NO:2).

The length of the protein is preferably at least 50 amino acids, more preferably at least 100 amino acids and e.g. up to 1000 or 500 amino acids. In a preferred embodiment of the invention, a side chain of the non-genetically encoded amino acids comprises a reactive group such as an alkyne, alkene, diene, (e.g. 1,3-diene), heterodiene (e.g. Michael systems such as alpha-beta unsaturated carbonyl or alpha-beta unsaturated heterocarbonyl compounds), hydrazine or hydroxylamino group group which is reacted with functional molecules comprising a functional moiety bound to a reaction partner for the reactive group on the amino acid side chain. The reaction partner on the functional molecule may e.g. be an azide, nitrile oxide, diazomethyl or aldehyde group.

Functional molecules may e.g. be selected from reporter molecules, e.g. dyes, particularly fluorescent dyes, and molecules carrying groups for modifying the characteristics, e.g. pharmacological, pharmacokinetic, pharmacodynamic and/or immunological characteristics of the respective polypeptides. Examples of functional molecules are hydroxyethyl starch (HES) molecules, polyethylene glycol molecules⁽²⁹⁾, toxophores, such as maytansine, doxorubicin, auristatin or calicheamicin⁽³⁰⁾ or metal chelators such as DOTA (1,4,7,10-tetraazacyclododecane-1,4,7,10-tetraacetic acid⁽³¹⁾. More preferably the functional molecules are glycan molecules comprising mono-saccharide, di-saccharide, oligo- or poly-saccharide moieties comprising, e.g. up to 1, 2, 3, 5, 10 or 50 saccharide units. Specific examples of glycan molecules comprise mannose, galactose and/or sialic acid units.

In a preferred embodiment the introduction of functional molecules comprises a reaction between a first unsaturated reactive group, e.g. an alkyne, alkene, diene or heterodiene group (present on the amino acid side chain), and a second 1,3-dipolar reactive group, e.g. an azide, nitrile oxide or diazomethyl group present on the functional molecule. In an especially preferred embodiment, the reactive group is an alkyne group, which may react under Cu-catalysis with a suitable 1,3-dipolar reaction partner, e.g. an azide group. In a further especially preferred embodiment the reactive group may be an alkene group, e.g. a constrained alkene group, which may react without Cu-catalysis with a second 1,3-dipolar reactive group, e.g. an azide or nitrile oxide group, as e.g. described in (19). In a still further preferred embodiment, the reactive group may be a hydrazine group, which may be reacted with an aldehyde group, e.g. the aldehyde group present on a glycan molecule.

The method of the present invention is suitable for the synthesis of single or multiple functionalized proteins, particularly single or multiple glycosylated proteins having e.g. 1, 2, 3, 4 or more, e.g. 6-8 functional groups attached to the protein backbone via the side chain of a non-genetically encoded amino acids, particularly via the side chain of a lysine derivative as described above. More preferably, the functional groups, e.g. the glycan molecules groups, are attached to the protein backbone via N-heterocyclic linkage groups, particularly via 1,2,3-triazole groups, which are e.g. formed by reacting an unsaturated group with an azide group.

According to the method of the invention, single or multiple functionalized, e.g. glycosylated proteins are provided having functional, e.g. glycan groups which are attached to the protein backbone via the side chains of non-genetically encoded amino acids, particularly via N-heterocyclic linkage groups, particularly via 1,2,3-triazole groups.

Moreover, the invention refers to a nucleic acid encoding a protein within the region encoding the open reading frame of said protein, wherein said nucleic acid molecules comprises an amber stop codon (UAG) at one or more positions where a genetically encoded amino acid is to be replaced by said non-genetically encoded acid.

The present invention allows the manufacture of proteins carrying single or multiple functional molecules such as glycan molecules, by enzymatic synthesis. The method comprises two steps, wherein the first step involves the incorporation of a non-genetically encoded amino acid at one or more predetermined positions into a protein by enzymatic synthesis, wherein the non-genetically encoded amino acid carries a side chain with a reactive group. The second step involves the reaction of the reactive group on the amino acid side chain with a functional molecule comprising a reaction partner for the reactive group. The resulting functionalized proteins are suitable, e.g. for diagnostic or even therapeutic applications such as the administration of therapeutic antibody molecules.

Furthermore, the invention is to be explained in greater detail by the Figures and Examples as shown hereinbelow.
**Fig. 1** Depiction of the alkyne and alkene modified lysine derivatives **1** and **2** used for amber suppression modification of Yellow Fluorescence Protein (YFP). Fluorescence pictures (overlay) of the YFP *E. coli* cells after incorporation of a: 0 stop codons, b: 1 stop codon, no amino acid **1** or **2** present, c: 1 stop codon, **1** present; d: 3 stop codons, **1** present, e: 1 stop codon, **2** present; f: 3 stop codons, **2** present.

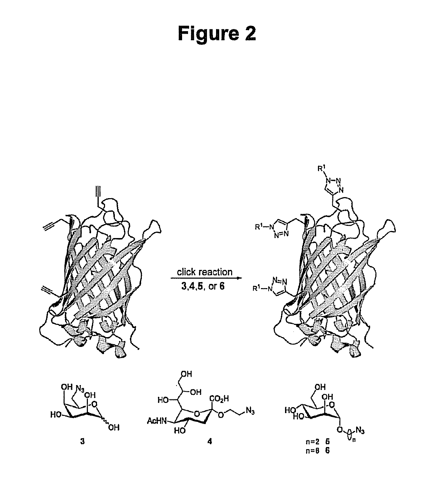

**Fig. 2** Depiction of the Cu-catalyzed click reaction with triple alkyne modified YFP and sugar azides 3-6.

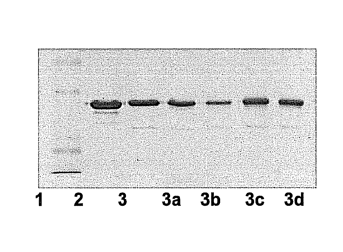

**Fig. 3** Click reaction of 1 mut-YFP. Lane 1: SeeBlue® Plus2 Prestained protein standard (*Invitrogen*); lane 2: wt-YFP; lane 3: 1 mut-YFP; lane 3a: click product of 1 mut-YFP and sugar azide **3;** lane 3b: click product of 1 mut-YFP and sugar azide **4;** lane 3c: click product of 1 mut-YFP and sugar azide **5;** lane 3d: click product of 1 mut-YFP and sugar azide **6.** The corresponding data of 3mut-YFP revealed similar results.

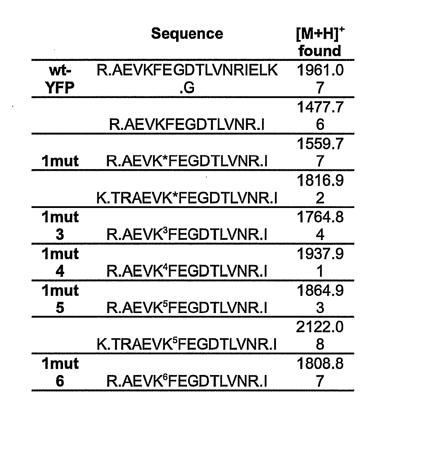

**Fig. 4** List of the allocated peptide fragments obtained after digest of the wt-YFP, mono-alkyne containing YFP (*) and click products of 1 mut YFP with azide **3-6** with trypsin (Protein sequence: SEQ ID NO:1: mvskgeelftgvvpilveldgdvnghkfsvsgegegdatygkltlkficttgklpvpwptlvttfgyglqcfary pdhmkqhdffksampegyvqertiffkddgnyktraev*fegdtlvnrielkgidfkedgnilghkleynyn shnvyimadkqkngikvnfkirhniedgsvqladhyqqntpigdgpvllpdnhylsyqsalskdpnekrd hmvllefvtaagitlgmdelykrsilkmasawshpqfeksg, (* = mutation of k to one alkyne).

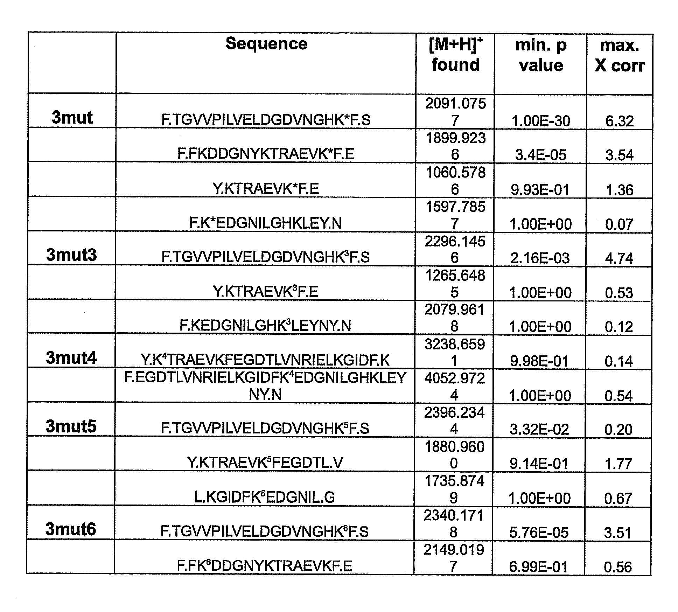

**Fig. 5** Analysis of peptide fragments of 3mut YFP and click products of 3mut YFP with sugar azides **3-6.** Protein sequence:SEQ ID NO:2: mvskgeelftgvvpilveldgdvngh*fsvsgegegdatygkltlkficttgklpvpwptlvttfgyglqcfary pdhmkqhdffksampegyvqertiffkddgnyktraev*fegdtivnrielkgidf*edgnilghkleynyn shnvyimadkqkngikvnfkirhniedgsvqladhyqqntpigdgpvllpdnhylsyqsalskdpnekrd hmvllefvtaagitlgmdelykrsilkmasawshpqfeksg, (* = mutation of k to three alkynes).

### Examples

### 1. Methods

Chemicals were purchased from *Sigma-Aldrich, Fluka* or *ACROS* and used without further purification. Solvents used were of reagent grade and purified by usual methods. Reactions were monitored on *Merck* Silica 60 F254 TLC plates. Detection was done by irradiation with UV light (254 nm) and staining with p-anisaldehyde solution in ethanol. Flash column chromatography was performed on Silica 60 *(Merck,* 230-400 mesh). NMR spectra were recorded on the following spectrometers: *Varian Oxford 200, Bruker AC 300, Varian XL 400* and *Bruker AMX 600.* The chemical shifts (δ) are given in ppm, the coupling constants (*J*) in Hz. Mass spectra were recorded on the following machines: Finnigan MAT 95 (EI), *Bruker Autoflex II* (MALDI-Tof) and Thermo Finnigan LTQ-FT (ESI-ICR).

### Synthesis and characterisation of alkyne 2

*N*_{α}-trifluoroacetyl-*L*-lysine-methylester **7**⁽²⁰⁾ and propargyl-pentafluorophenyl-carbonate **8** ⁽²¹⁾were prepared according to literature procedures.

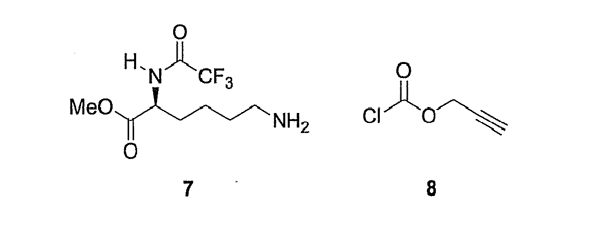

### (S)-2-(trifluoroacetylamino)-6-((prop-2-ynyloxy)carbonylamino)hexanoic-acid-methylester 9

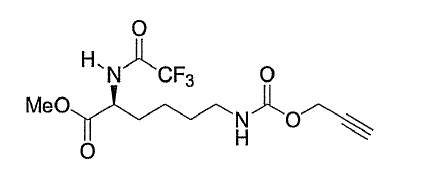

A solution of *N*_{α}-trifluoroacetyl-*L*-lysine methyl ester **7** (2.57 g, 10.00 mmol) and propargyl-pentafluorophenyl carbonate **8** (2.76 g, 11.00 mmol) in anhydrous DMF (16 mL) was cooled to -10°C and anhydrous pyridine (1.78 mL, 22.00 mmol) was added dropwise. The solution was allowed to attain RT slowly and was stirred overnight. The reaction mixture was diluted with CH₂Cl₂, washed with 0.5 M HCl, twice with H₂O and brine. The organic phase was dried over Na₂SO₄ and evaporated in vacuo. Flash column chromatography (SiO₂, *i*Hex/EtOAc 10:1→1:2) afforded the title compound **9** as a colorless oil (2.85 g, 84%).

R_{f} = 0.71 (*i*Hex/EtOAc 2:1); ¹H-NMR (600 MHz, CDCl₃): δ = 7.14 (*bs*, 1H, NH), 4.91 (*bs*, 1H, NH), 4.66 (*m*, 1H, C*H*₂), 4.57 (*dd*, J=12.5 Hz, J=7.5 Hz, 1H, C*H*), 3.78 (s, 3H, OC*H*₃), 3.12-3.24 (*m*, 2H, C*H*₂), 2.46 (*t*, J=2.4 Hz, 1H, C*H*), 1.90-1.98 (*m*, 1H C*H*₂), 1.73-1.86 (*m,* 1H C*H*₂), 1.47-1.59 (*m*, 2H, C*H*₂), 1.27-1.44 (*m*, 2H, C*H*₂), ppm; ¹³C-NMR (150 MHz, CDCl₃): δ = 171.4 (*C*O), 157.2 (*C*O), 156.0 (*C*O), 115.8 (*C*F₃), 78.4 (*C*), 74.8 (*C*H), 53.1 (CH₃), 52.8 (*C*H), 52.7 (*C*H₂), 40.4 (*C*H₂), 31.3 (*C*H₂), 29.6 (*C*H₂), 22.1 (*C*H₂) ppm; HR-MS (ESI, pos.) *m*/*z* calcd. for C₁₃H₁₈O₅N₂F₃⁺ [M+H]⁺: 339.1162, found 339.1152.

### (S)-2-Amino-6-((prop-2-ynyloxy)carbonylamino)hexanoic acid Li salt 1

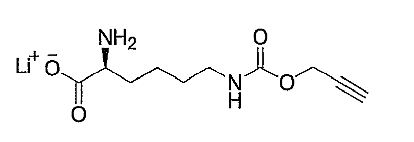

To a solution of **9** (1.59 g, 4.69 mmol) in THF/MeOH/H₂O (2:2:1, 65 mL) was added LiOH*H₂O (0.59 g, 14.00 mmol) and the reaction mixture was stirred at RT for 4 h. The reaction was filtered and the filtrate evaporated in vacuo. Flash column chromatography (RP-C18, H₂O→H₂O/MeOH 10:1) followed by lyophylization afforded the title compound **1** as a white solid (998 mg, 91%).
¹H-NMR (400 MHz, D₂O): δ = 4.69 (*m*, 2H, C*H*₂), 3.27 (*m*, 1H, C*H*), 3.17 (*m*, 2H, C*H*₂), 1.48-1.73 (*m*, 4H, 2xC*H*₂), 1.30-1.44 (*m*, 2H, C*H*₂), ppm; ¹³C-NMR (100 MHz, CDCl₃): δ = 183.3 (*C*O), 157.6 (*C*O), 78.1 (*C*), 75.3 (*C*), 55.8 (*C*H), 52.6 (*C*H₂), 40.27 (*C*H₂), 34.1 (*C*H₂), 29.7 (*C*H₂), 22.1 (*C*H₂) ppm; HR-MS: (ESI, pos.) *m*/*z* calcd. for C₁₀H₁₆O₄N₂⁺ [M]⁺: 228.1110, found 228.1105.

### Synthesis of sugar azides

Galactose-azide **3**⁽¹⁴⁾ and 2-azidoethyl-N-acetylneuraminic acid **4**⁽²²⁾ were synthesized as reported in the literature.

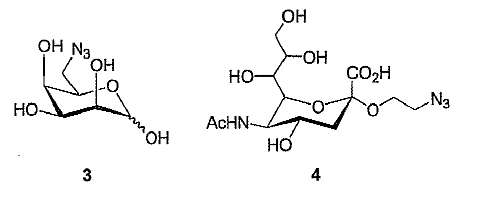

### 2'-Bromo-ethyl-α-D-2,3,4,6-tetra-O-acetylmannopyranose 10⁽²³⁾

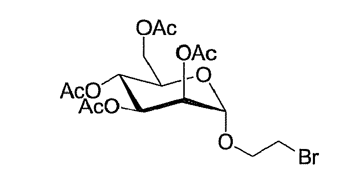

To an ice bath cooled solution of peracetylated D-mannose (1.84 g, 4.72 mmol) and 2-bromoethanol (0.4 mL) in anhydrous CH₂Cl₂ (10 mL) was added dropwise BF₃Et₂O (2.9 mL). The reaction mixture was stirred for 1 h at 0°C and further 16 h at RT. The mixture was poured into ice-water (10 mL), the aqueous phase was extracted with CH₂Cl₂ twice (10 mL) and the combined organic phases were washed with water, saturated NaHCO₃ solution and water, dried over Na₂SO₄, filtered and concentrated in vacuo. Flash column chromatography (SiO₂, *i*Hex/EtOAc 3:1) yielded **10** as a white amorphous solid (1.51 g, 70 %).

R_{f} = 0.51 (*i*Hex/EtOAc 1:1); ¹H-NMR (400 MHz, CDCl₃) δ = 5.34 (*dd*, J=10.1 Hz, J=3.4 Hz, 1H, H-3), 5.30-5.25 (*m*, 2H, H-4, H-2), 4.86 (*d*, J=1.7 Hz, 1H, H-1), 4.26 (*dd*, J=12.7 Hz, J=6.0 Hz, 1H, H-6a), 4.15-4.10 (*m*, 2H, H-5, H-6b), 3.97 (*dt,* J=11.2 Hz, J=6.3 Hz, 1H, CH*H*CH₂Br), 3.88 (*dt*, J=11.3 Hz, J=5.8 Hz, 1H, C*H*HCH₂Br), 3.51 (*t*, J=6.0 Hz, 2H, C*H*₂Br), 2.15 (s, 3H, C*H*₃CO₂), 2.10 (s, 3H, C*H*₃CO₂), 2.04 (s, 3H, C*H*₃CO₂), 1.99(s, 3H, C*H*₃CO₂) ppm; ¹³C-NMR (100 MHz, CDCl₃) δ = 170.8 (CH₃*C*O₂), 170.2 (CH₃*C*O₂), 170.0 (CH₃*C*O₂), 169.9 (CH₃*C*O₂), 98.0 (C-1), 69.6 (C-2), 69.3 (C-3), 69.1 (C-5), 68.7 (*C*H₂CH₂Br), 66.2 (C-4), 62.6 (C-6), 29.8 (*C*H₂Br), 21.0 (2C, *C*H₃CO₂), 20.9 (2C, CH₃CO₂) ppm; HRMS (ESI, pos.) *m*/*z* calcd. for C₁₆H₂₇BrNO₁₀⁺ [M+NH₄]⁺ 272.0813, found 272.0814.

### 2'-Azidoethyl-a-D-2,3,4,6-tetra-O-acetylmannopyranose 11⁽²⁴⁾

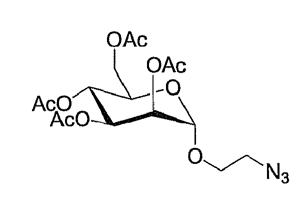

**10** (0.91 g, 2.00 mmol) and tetrabutylammonium bromide (0.065 g, 0.20 mmol) were dissolved in anhydrous DMF (6 mL) and sodium azide (0.26 g, 4.0 mmol) was added. After stirring overnight at RT the mixture was poured into water (20 mL). The aqueous phase was extracted twice with EtOAc, the combined organic phases were dried over MgSO₄, filtered and concentrated in vacuo. Flash column chromatography (SiO₂, *i*Hex/EtOAc 3:1) yielded **11** as a colorless solid (736 mg, 88 %).

R_{f} = 0.46 (*i*Hex/EtOAc 1:1); ¹H-NMR (600 MHz, CDCl₃) δ = 5.35 (*dd*, J=10.0 Hz, J=3.5 Hz, 1H, H-3), 5.29 (*t*, J=10.0 Hz, 1H, H-4), 5.28 (*dd*, J=3.4 Hz, J=1.7 Hz, 1H H-2), 4.86 (*d*, J=1.7 Hz, 1H, H-1), 4.28 (*dd*, J=12.3 Hz, J=5.4 Hz, 1H, H-6a), 4.12 (*dd*, J=12.3 Hz, J=2.4 Hz, 1H,H-6b), 4.04 (*ddd,* J=9.9 Hz, J=5.3 Hz, J=2.4 Hz, 1H, H-5), 3.86 (*ddd,* J=10.7 Hz, J=7.0 Hz, J=3.7 Hz, 1H, CH*H*CH₂N₃), 3.67 (*ddd,* J=10.6 Hz, J=6.0 Hz, J=3.6 Hz, 1H, C*H*HCH₂N₃), 3.49 (*ddd*, J=10.6 Hz, J=7.0 Hz, J=3.6 Hz, 1H, CH₂CH*H*N₃), 3.43 (*ddd*, J=13.3 Hz, J=6.0 Hz, J=3.7 Hz, 1H, CH₂C*H*HN₃) ppm; ¹³C-NMR (150 MHz, CDCl₃) δ = 170.8 (CH₃*C*O₂), 170.2 (CH₃*C*O₂), 170.0 (CH₃*C*O₂), 169.9 (CH₃*C*O₂), 97.9 (C-1), 69.6 (C-2), 69.1 (C-3), 69.0 (C-5), 67.3 (*C*H₂CH₂N₃), 62.7 (C-6), 50.6 (*C*H₂N₃), 21.1 (CH₃*C*O₂), 21.0 (2C, *C*H₃CO₂), 20.9 (*C*H₃CO₂) ppm; HRMS (ESI, pos.) *m*/*z* calcd. for C₁₆H₂₇N₄0₁₀⁺ [M+NH₄]⁺ 435.1722, found 435.1722.

### 2'-Azidoethyl-α-D-2,3,4,6-tetra-O-acetylmannopyranose 5

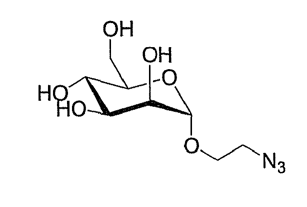

Sugar **11** (0.71 g, 1.70 mmol) was dissolved in anhydrous MeOH (8 mL) and a solution of NaOMe in MeOH (1 M, 0.35 mL) was added. After stirring for 2.5 h at RT the solvent was removed under reduced pressure. After flash column chromatography (SiO_{2,} CH₂Cl₂/MeOH 10:1→6:1) the title compound **5** was obtained as a colorless solid (377 mg, 89 %).

R_{f} = 0.13 (CH₂Cl₂/MeOH 10:1); ¹H-NMR (400 MHz, CD₃OD) δ = 4.82 (*d*, J=1.7 Hz, 1H, H-1), 3.95-3.88 (*m*, 1H, C*H*HCH₂N₃), 3.87-3.82 (*m*, 2 H, H-6a, H-2), 3.75-3.69 (*m*, 2H, H-6b, H-4), 3.66-3.54 (*m*, 3 H, CH*H*CH₂N₃, H-3, H-5), 3.41 (*t*, J=5.0 Hz, 2H, CH₂C*H*₂N₃) ppm; ¹³C-NMR (100 MHz, CD₃OD) δ = 101.9 (C-1), 75.0 (C-5), 72.6 (C-4), 72.2 (C-2), 68.7 (C-3), 67.8 (*C*H₂CH₂N₃), 63.0 (C-6), 51.9 (CH₂*C*H₂N₃) ppm; HRMS (ESI, pos.) *m*/*z* calcd. for C₈H₁₅N₃O₆Na⁺ [M+Na]⁺ 272.0853, found 272.0854.

### 2,3,4,6-Tetraacetylmannopyranosyl-α-1-O-trichloracetimidate 12

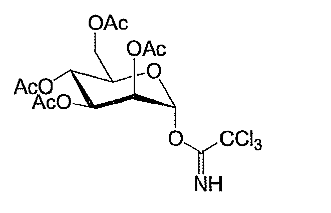

After heating a solution of peracetylated D-mannose (3.75 g, 10.02 mmol) in DMF (35 ml) to 50°C hydrazine hydrate (80% in water, 0.61 g, 12.14 mmol, 0.59 mL) was added and stirred for 45 min at 50°C. The reaction mixture was poured on water and the aqueous phase was extracted twice with EtOAc. The combined organic phases were dried over MgSO₄, filtered and the solvent was removed in vacuo. The obtained crude product was immediately dissolved in anhydrous CH₂Cl₂ (35 mL) under nitrogen at room temperature and Cs₂CO₃ (3.90 g, 11.97 mmol) was added. After stirring for 15 min trichloroacetonitrile (10.00 mL, 99.73 mmol) was added dropwise to the dark red suspension and stirring was continued for further 24 h in which time the reaction mixture turned dark-brown. The suspension was filtered through a thin layer of Celite and the solvent was removed in vacuo. Purification via flash column chromatography (SiO₂, *i*Hex/EtOAc 2:1) obtained **12** (1.11 g, 23 % over two steps) as a colorless foam.

R_{f} = 0.46 (*i*Hex/EtOAc 1:1); ¹H-NMR(300 MHz, CDOl₃) δ = 8.77 (s, 1H, N*H*), 6.23 (*d*, J=1.9 Hz, 1H, H-1), 5.41 (*dd*, J=3.1 Hz, J=1.9 Hz, 1H, H-2), 5.37-5.32 (*m*, 2H, H-3, H-4), 4.27-4.07 (*m*, 3H, H-5, H-6a, H-6b), 2.14 (s, 3H, C*H*₃CO₂), 2.03 (s, 3H, C*H*₃CO₂), 2.01 (s, 3H, C*H*₃CO₂), 1.95 (s, 3H, C*H*₃CO₂) ppm; ¹³C-NMR (75 MHz, CDCl₃) δ = 170.6 (CH₃*C*O₂), 169.8 (CH₃*C*O₂), 169.7 (2C, CH₃*C*O₂), 159.7 (*C*NH), 94.6 (C-1), 90.6 (*C*Cl₃), 71.3 (C-5), 68.9 (C-3), 67.9 (C-2), 65.5 (C-4), 62.1 (C-6), 20.8 (*C*H₃CO₂), 20.7 (3C, *C*H₃CO₂) ppm; HRMS (ESI, pos.) *m*/*z* calcd. for C₁₄H₂₄NO₁₀⁺ [M+NH₄]⁺: 366.1395, found 366.1398.

### 6'-Azidohexyl-α-D-mannopyranose 6

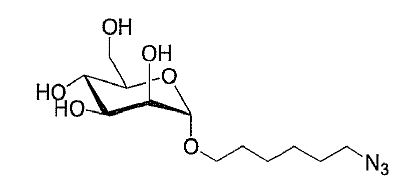

To a solution of **12** (0.48 g, 0.97 mmol) and 6-azidohexan-1-ol (0.29 g, 2.05 mmol) in anhydrous CH₂Cl₂ (9 mL) under Ar was added 3A molecular sieves. The suspension was stirred for 1 h at RT and BF₃ Et₂O (0.1 mL) was added at 0°C. After stirring overnight the mixture was neutralized with NEt₃, diluted with CH₂Cl₂, filtered through Celite and the filtrate was concentrated. Flash column chromatography (SiO_{2,} *i*Hex/EtOAc 3:1) of the residue yielded a mixture of the immediate product and 6-azidohexan-1-ol. The mixture was dissolved in anhydrous methanol (2 mL). A solution of NaOMe in methanol (0.1 ml) was added, and the mixture was stirred for 3 h at RT. After removal of the solvent the residue was subjected to flash column chromatography (CH₂Cl₂/MeOH 15:1). The title compound **6** was obtained as colorless oil (87 mg, 29 %).

R_{f} = 0.34 (CH₂Cl₂/MeOH 10:1); ¹H-NMR (400 MHz, CDCl₃) δ = 4.74 (*d*, J=1.7 Hz, 1H, H-1), 3.82 (*dd*, J=11.7 Hz, J =2.4 Hz, 1H, C-6a), 3.78 (*dd*, J=3.3 Hz, J=1.7 Hz, 1H, H-2), 3.77-3.66 (*m*, 3H, OCH*H*CH₂, H-3, H-6b), 3.61 (*t*, J=9.5 Hz, 1H, H-4), 3.52 (*ddd,* J=9.6 Hz, J=5.7 Hz, J=2.4 Hz, 1H, H-5), 3.43 (*dt,* J=9.6 Hz, J=6.2 Hz, 1H, OC*H*HCH₂), 3.31-3.26 (*m*, 2H, C*H*₂N₃), 1.67-1.53 (*m*, 4 H), 1.48-1.36 (*m*, 4 H) ppm; ¹³C-NMR (100 MHz, CDClₛ) δ = 101.7 (C-1), 74.7 (C-5), 72.8 (C-3), 72.4 (C-2), 68.8 (C-4), 68.5 (O*C*H₂(CH₂)₅N₃), 63.1 (C-6), 52.5 (*C*H₂N₃), 30.6 (*C*H₂), 30.0 (*C*H₂), 27.7 (*C*H₂), 27.1 (*C*H₂) ppm; HRMS (ESI, pos.) *m*/*z* calcd. for C₁₂H₂₇N₄O₆⁺ [M +NH₄]⁺ 323.1925, found 323.1927.

### Expression and purification of YFP

### Cloning procedure

Wildtype *yfp* gene was cloned in a two-step Gateway@ reaction via the vector pDONR201 (Invitrogen) into the expression vector pDEST007⁽²⁵⁾, which results in the addition of an N-terminal *Strep-Tag II* (*IBA*).

Synthetic *Mm pylT* gene was purchased from *Metabion* and inserted into the first multiple cloning site of pET-Duet-1^{™} vector (*Novagen*) using the Notl and AfIII restriction sites. *Mm pylS* was PCR-amplified from genomic DNA using *Phusion HF* DNA polymerase (*Finnzymes*) with the two primers 5'-GGA TCC CAT GGA TAA AAA ACC ACT AAA CAC TC-3' (SEQ ID NO:3) and 5'-GCG GCC GCT TAC AGG TTG GTA GAA ATC CCG-3' (SEQ ID NO: 4). The PCR product was added, via the pCR®2.1-TOPO® vector (*Invitrogen*), into the first multiple cloning site of pET-Duet1+ *Mm* pylT between the restriction sites BamHI and Notl, generating the vector pET-Duet1+ Mm pylST. Constitutive PylS and PylT production was allowed by exchange of T7 promoter-1 of pET-Duet1+ *Mm* pylST with a synthetic *E.coli* Trp-Promoter (*Metabion*) using the restriction sites Clal and Xbal (pTRP-Duet1+ *Mm* pylST). Mutant variants of yfp were generated via QuickChange® Site-Directed Mutagenesis Kit (*Stratagene*) using the primers 5'-GCG CCG AGG TGT AGT TCG AGG GCG ACA CCC-3' (SEQ ID NO:5) and 5'-GGG TGT CGC CCT CGA ACT ACA CCT CGG CGC-3' (SEQ ID NO: 6) for *1mu- yfp,* additionally the primers 5'-CGT AAA CGG CCA CTA GTT CAG CGT GTC CG-3' (SEQ ID NO:7) and 5'-CGG ACA CGC TGA ACT AGT GGC CGT CTA CG-3' (SEQ ID NO:8) for *2mut-yfp,* and additionally the primers 5'-GCA TCG ACT TCT AGG AGG ACG GCA ACA TCC TGG GGC ACA AGC TGG-3' (SEQ ID NO:9) and 5'-CCA GCT TGT GCC CCA GGA TGT TGC CGT CCT CCT AGA AGT CGA TGC-3' (SEQ ID NO:10) for *3mut-*yfp. Mutant yfp variants were PCR-amplified with *Phusion HF* DNA polymerase (*Finnzymes*) and the two primers 5'-CAT ATG ATG GTG AGC AAG GGC G-3' (SEQ ID NO:11) and 5'-GGT ACC TTA TCC GGA TTT TTC GAA TTG AGG ATG ACT CCA TGC GCT AGC CAT CTT AAG GAT AGA TCT CTT GTA CAG-3' (SEQ ID NO:12), resulting in a C-terminal *Strep-Tag II*. PCR products were cloned, via the pCR4®-Blunt TOPO® vector (*Invitrogen*), into the second multiple cloning site of pTRP-Duet1+ Mm pylST.

### Expression and purification of YFP

Wildtype YFP was overexpressed in *E.coli* strain Rosetta-gami^{™} 2. Cells were cultivated (37°C, 200 rpm) in LB-medium until OD₆₀₀ = 0.6. Protein expression was induced by addition of 200 µg anhydrotetracycline.

*E.coli* strain BL21 (DE3) was used for the production of mutant YFP variants. Cells were grown in LB-medium containing 2 mM amino acid **1** or **2** (37°C, 200 rpm) until OD₆₀₀ = 0.6. Protein expression was induced by addition of 1 mM IPTG. After further 4 h incubation cells were harvested (10.000 x g, 8 min, 4°C) and stored at -20°C. All purification steps were carried out at 4°C. Cells were resuspended in StrepA buffer (*GE Healthcare*) (10 mM Tris-HCI pH 8, 1 mM EDTA, 150 mM NaCI) supplemented with protease inhibitor mix (*Roche*). The crude cell extract was solubilized in a French press (*Thermo Scientific*). Cell debris was removed by centrifugation (38 000x g, 30 min, 4°C) and the supernatant was applied to a 5 ml Strep-Tactin column (*IBA*), equilibrated with StrepA buffer. Proteins were eluted from the column using the same buffer containing 2.5 mM desthiobiotin. The eluted fractions were pooled, concentrated and loaded onto a 1 mL MonoQ column (GE Healthcare) equilibrated with YFP storage buffer (10 mM Tris-HCI pH 8, 10 mM EDTA). The protein was eluted with a linear gradient of 25 column volumes with the same buffer containing 1 M NaCl. Purified YFP was concentrated and stored at -80°C.

### Fluorescence microscopy

After protein expression cells were monitored on a *TCS SPE* spectral confocal microscope (Leica) with an inverse stand and the HCX FL APO 63x/140-0.60 oil objective (excitation: 488 nm).

### Bioconjugation via click chemistry with sugar azides 3-6

The Cu-catalyzed click reaction was performed in YFP storage. The protein solution (2.20 µL, 3 µg, 0.1 nmol) was premixed with the corresponding azide (0.5 µL, 0.1 M in DMSO, 50.0 nmol) and diluted with YFP storage buffer (1.9 µL). A freshly premixed solution of CuSO₄ (0.1 µL, 0.1 M in H₂O, 10.0 nmol) sodium-ascorbate (0.1 µL, 0.1 M in H₂O, 10.0 nmol) and TBTA ligand^{[26]} (tris-(benzyltriazolylmethyl)amine, 0.2 µL, 0.1 M in DMSO, 20.0 nmol) was carefully added. The mixture was vortexed and shaken at RT overnight. The reaction mixtures were used for PAGE analysis and tryptic digestions without further purifications.

### Gel electrophoreses

SDS-PAGE of proteins was performed on the MiniProtean® 3 Cell system (*Bio-Rad*) under standard conditions. Protein bands were visualized with standard Coomassie Blue staining.

### Tryptic digestion

Sample preparation for mass spectrometry was attained via standard tryptic digestion. The Coomassie Blue stained SDS-gel was washed twice for 10 min with water (bidest.). Protein bands were excised from the gel, cut in small cubes (ca. 1mm³) and transferred to eppendorf-cups. The gel particles were washed with water (100 µL) for 15 min and 50 mM NH₄HCO₃/acetonitrile (each 100 µL) for further 15 min. All liquids were removed and the gel cubes covered with acetonitrile (100 µL). After removal of acetonitrile the gel particles were rehydrated with 50 mM NH₄HCO₃ (100 µL). After 5 min 100 µL acetonitrile were added and washed for further 15 min. The liquids were removed and again 100 µL acetonitrile added to shrunk the particles whereas after 5 min all acetonitrile was removed. The gel cubes were dried at RT for 10 min.

Protein denaturation and reduction were carried out by adding DTT (10 mM in 50 mM NH₄HCO₃, 100 µL) and incubation for 45 min at 56°C. The tubes were chilled to RT, all liquids were removed and alkylation was performed by addition of iodoacetamide (55 mM im 50 mM NH₄HCO₃, 100 µL). Incubation for 30 min at RT (in the dark) and removal of all liquids were followed by further washing steps (15 min at RT with 50 mM NH₄HCO₃,/acetonitrile, each 100 µL, twice). Further addition of acetonitrile (100 µL) resulted in shrinkage of the gel cubes which was completed by removal of all liquids and drying in a vacuum centrifuge (10 min). In-gel digestion was carried out by rehydration of the gel particles in 100 µL 25 mM NH₄HCO₃, addition of trypsin or chrymotrypsin (1 µg, *Promega*) and incubation at 37°C overnight.

All reaction solution was taken and stored separately. For the extraction of peptides the gel particles were resuspended in 100 µL 25 mM NH₄HCO₃ and sonificated. After 15 min acetonitrile (100 µL) was added and the reaction mixture again sonificated for 15 min. All peptide solution was removed and stored separately. Additional extraction steps were achieved by sonification with 5% formic acid and acetonitrile (each 100 µL for 15 min). Finally all peptide solutions were combined, concentrated in a vacuum centrifuge (down to 20 µL) and used without further treatment for peptide mass analysis.

### Peptide mass spectrometry

For the nano-HPLC ESI mass spectrometry analysis the (chymo)tryptic digested peptides were loaded onto a Dionex C18 Nano Trap Column (100 µm), subsequently eluted and separated by a Dionex C18 PepMap 100 (3 µm) column for analysis by tandem MS followed by HR-MS using a coupled Dionex Ultimate 3000 LC-ThermoFinnegan LTQ-FT MS system.

The obtained data of peptide fragments were searched using the SEQUEST algorithm against *Aequoria victoria* YFP (1F0B_A, GI:11513674) via the software "Xcalibur bioworks". The search was limited to (chymo)tryptic peptides, two missed cleavage sites, monoisotopic precursor ions and a peptide tolerance of <10 ppm.

### 2. Results and Discussion

We found that the propargyl-protected Lys derivative 1 and the commercially available alloc-Lys derivative 2 were adequately efficient substrates for the pyrrolysine tRNA synthetase.

In order to incorporate them into a protein we transformed *E. coli* with a vector containing the genes for the Mm pyrrolysine tRNA synthetase, the Mm tRNA_{CUA}, a C-terminal Strep-tag modified, yellow fluorescent protein (YFP) with initially one later three amber TAG stop codons at the positions 27, 114, and 132 as well as an ampicilline resistance gene. We added the alkyne amino acid 1 and the alkene amino acid 2 to the medium containing the stably transformed growing *E. coli* cells. As depicted in Fig. 1, the *E. coli* cells containing the wild type YFP (Fig 1a) are strongly fluorescent showing that the YFP protein is efficiently produced by the bacteria. *E. coli* cells containing the YFP protein with one TAG-stop codon (Fig. 1 b) in the absence of the alkyne or alkene amino acids 1 or 2 are non fluorescent showing that in this case the intact protein can not be manufactured. Figs. 1 c and 1 d show *E. coli* cells containing the YFP protein with one and three TAG stop codons in the presence of the alkyne amino acid 1 (data for amino acid 2 are similar). Here fluorescent cells are clearly detected showing that the stop codons are suppressed in the presence of the artificial amino acids in the medium. The fluorescence in Fig. 1d is clearly weaker indicating that although suppression of three stop codons is possible, the total suppression efficiency decreases. In order to quantify the protein yield we isolated the Strep-tagged YFP proteins from 1 L *E. coli* cultures and determined the yield of about 0.3 mg for the YFP proteins comprising one alkyne or alkene amino acid. For comparison we also quantified the amount of wild type protein from the analyzed 1 L cultures to about 2 mg protein. These data show that the whole suppression procedure reduces the protein yield by a factor of 10 in our hands. To our surprise we were able to isolate 30 µg of the pure triple alkyne modified YFP from the corresponding 1 L cell culture. This may look small but for the first reported incorporation of three encoded artificial amino acids into a protein the amount is substantial and promising.

We next wanted to proof the presence of the alkyne amino acids particularly in the triple modified YFP protein to exclude that the *E. coli* cells found a way e.g. to repair or to exchange the stop codons in the triple AUG containing YFP gene. In addition we intended to investigate the ability to use the alkyne and alkene units for the connection of different sugar units. As click reactions with alkyne amino acids are better worked out we performed the experiments with the alkyne modified proteins. The purified YFP proteins containing one and three alkyne units were therefore treated with galactose and mannose azides 3, 5, and 6 as well as a solution of CuSO₄, sodium ascorbate and TBTA ligand. In addition we used the biologically most relevant sialic acid derivative 4 shown in Fig. 2. The results of an SDS-PAGE gel-electrophoresis of the proteins after click-reaction is shown in Fig. 3 After incubation of the protein click mixture overnight we digested the click-modified proteins with trypsin or chymotrypsin to generate peptides produced by enzymatic cuts behind basic (trypsin) and aromatic (chymotrypsin) amino acids. The various peptide fragments of the digests were analyzed by HPLC-MS/MS using a high resolution Orbitrap mass spectrometer and compared to the theoretically expected peptides with the software "Xcalibur bioworks". Figs. 4 and 5 contain lists of the clearly allocated peptides in proteins 1 mut YFP and 3 mut YFP. Most important is that the mass spectrometer found the expected sugar modified peptides in all cases proofing that the three stop codons are not only present at the expected sites but that they were efficiently glycosylated at the protein using click chemistry.

### List of References

- [1]: B.G. Davis, Angew.Chem.lnt.Ed. 2009, 48, 4674-4678
- [2]: S.J. Danishefsky, Gordon Research Conference on Natural Products, 2005
- [3]: E.L. Decker, R. Reski, Bioprocess. Biosyst.Eng. 2008, 31, 3-9
- [4]: K. Ko, M.H. Ahn, M. Song, Y.K. Choo, H.S. Kim, K. Ko, H. Joung, Mol.Cells, 2008, 25, 494-503
- [5]: N. Sethuraman, T.A. Stadheim, Curr.Opin.Biotechnol. 2006, 17, 341-346
- [6]: P.E. Dawson, S.B. Kent, Annu.Rev.Biochem. 2000,69,923-960
- [7]: S. Mezzato, M. Schaffrath, C.Unverzagt, Angew.Chem.Int.Ed. 2005,44,1650-1654
- [8]: T.W. Muir, Annu.Rev.Biochem. 2003, 72, 249-289
- [9]: B.L. Nilsson, M.B. Soellner, R.T. Raines, Annu.Rev.Biophys. Biomol.Struct. 2005,34,91-118
- [10]: Y.Shin, K.A. Winans, B.J. Backes, S.B. Kent, J.A. Ellman, C.R. Bertozzi, J.Ann.Chem.Soc. 1999,121,11684-11689
- [11]: A.J. de Graaf, M. Kooijman, W.E. Hennink, E. Mastrobattista, Bioconjug.Chem. 2009,20, 1281-1295
- [12]: V.V. Rostovtsev, L.G. Green, V.V. Fokin, K.B. Sharpless, Angew. Chem. 2002,114,2708-2711; Angew. Chem. Int. Ed., 2002, 2741, 2596-2599
- [13]: C.W. Tornoe, C. Christensen, M. Meldal, J.Org.Chem. 2002, 67, 3057-3064
- [14]: G.A. Burley, J. Gierlich, M.R. Mofid, H. Nir, S. Tal, Y. Eichen, T. Carell, J.Am.Chem.Soc. 2006,128,1398-1399
- [15]: S.W. Santoro, L. Wang, B. Herberich, D.S. King, P.G. Schultz, Nat.Biotechnol. 2002,20, 1044-1048
- [16]: T. Fekner, X. Li, M.M. Lee, M.K. Chan, Angew.Chem.Int.Ed. 2009,48, 1633-1635
- [17]: D.P. Nguyen, H. Lusic, H. Neumann, P.B. Kapadnis, A. Deiters, [18] J.W. Chin, J.Am.Chem.Soc. 2009,131,8720-8721 T. Yanagisawa, R. Ishii, R. Fukunaga, T. Kobayashi, K. Sakamoto, S. Yokoyama, Chem.Biol. 2008,15,1187-1197
- [19]: K. Gutsmiedl, C.T. Wirges, V. Ehmke, R. Carell, Org.Lett. 2009,11, 2405-2408
- [20]: B. Hao, G. Zhao, P.T. Kang, J.A. Soares, T.K. Ferguson, J. Gallucci, J.A. Krzycki, M.K. Chan, Chem.Biol. 2004, 11, 1317- 1324
- [21]: R. Ramesh, S. Rajasekaran, R. Gupta, S. Chandrasekaran, Org.Lett. 2006,8, 1933-1936
- [22]: X.L. Sun, Y. Kanie, C.T. Guo, O. Kanie, Y. Suzuki, C.H. Wong, Eur.J.Org.Chem. 2000, 2643-2653
- [23]: J. Dahmen, T. Frejd, G. Gronberg, T. Lave, G. Magnusson, G. Noori, Carbohydr.Res. 1983,116, 303-307
- [24]: M. Kleinert, N. Röckendorf, T.K. Lindhorst, Eur.J.Org.Chem. 2004, 3931-3940
- [25]: M. Ober, H. Müller, C. Pieck, J. Gierlich, T. Carell, J.Am. Chem. Soc. 2005,127, 18143-18149
- [26]: Q. Wang, T.R. Chan, R. Hilgraf, V.V. Fokin, K.B. Sharpless, M.G. Finn, J.Am.Chem.Soc. 2003,125, 3192-3193
- [27]: R. Xu, S.R. Hanson, Z. Zhang, Y.-Y. Yang, P.G.Schultz and C.- H. Wong, J.Am.Chem.Soc.,2004.126,15654-15655
- [28]: *J.AmChem.Soc.,* Additions & Corrections JA906705A
- [29]: Kang J.S., Deluca P.P., Lee K.C., Expert Opin Emerg Drugs., 2009, Jun; 14(2):363-80
- [30]: Wu A.M, Senter P.D., Nat.Biotechnol.,2005,Sep;23(9):1137-46
- [31]: Friodevaux S., Eberle A.N., Biopolymers,2002; 66(3):161-83

## Claims

1. A method of incorporating a non-genetically encoded amio acid into a protein, comprising:
(a) providing a nucleic acid molecule encoding said protein, wherein said nucleic acid molecule comprises an amber stop codon (UAG) at one or more positions within the region encoding the open reading frame of said protein where a genetically encoded amino acid is to be replaced by said non-genetically encoded acid,
(b) expressing said nucleic acid molecule in an expresion system capable of amber codon suppression, in the presence of said non-genetically encoded amino acid under conditions wherein the non-genetically encoded amino acid is incorporated into said protein at said one or more postions, and
(c) recovering the expression product.

2. The method of claim 1,
wherein said non-genetically encoded amino acid comprises a side chain with a reactive group, e.g. an alkyne, alkene, diene, heterodiene, hydrazine or hydroxyamine group.

3. The method of claim 1 or 2,
wherein said non-genetically encoded amino acid is a modified lysine derivative.

4. The method of any one of claims 1-3,
wherein the expression system comprises a prokaryotic host cell, particularly *E.coli*, or a eukaryotic host cell.

5. The method of any one of claims 1-4,
wherein the expression system comprises an amber codon suppressing tRNA synthetase/tRNA_{CUA} pair.

6. The method of claim 5, wherein the expression system comprises an amber codon suppressing pyrrolysyl tRNA synthetase/tRNA_{CUA} pair, particularly a *Methanosarcina* tRNA synthetase/tRNA_{CUA} pair.

7. The method of any one of claims 1-6,
wherein the number of amino acid replacement positions is 1, 2, 3 or 4.

8. The method of any one of claims 1-7,
further comprising:
(d) coupling a functional molecule to the side chain of said non-genetically encoded amino acid.

9. The method of claim 8,
wherein the functional molecule is selected from reporter molecules, glycan molecules, hydroxyethyl starch molecules, polyethylene glycol molecules, toxophores or metal chelators.

10. The method of claims 8 or 9, wherein the coupling of the functional molecule comprises a reaction between a first unsaturated reactive group, e.g. an alkyne, alkene, diene or heterodiene group, and a second 1,3-dipolar reactive group, e.g. an azide, nitrile oxide or diazomethyl group.

11. Use of a method of any one of claims 1-10 for the synthesis of single or multiple functionalized, e.g. glycosylated proteins.

12. A single or multiple functionalized, e.g. glycosylated protein, wherein the functional, e.g. glycan groups, are attached to the protein backbone via the side chain of a non-genetically encoded amino acid.

13. The protein of claim 12, which has 1, 2, 3 or 4 functionalized, e.g. glycosylated positions.

14. The protein of claim 12 or 13,
wherein the functional groups are attached to the protein backbone via N-heterocyclic linkage groups, particularly via 1,2,3-triazole groups.

15. A nucleic acid molecule encoding a protein, wherein said nucleic acid molecule comprises an amber stop codon (UAG) at one or more positions within the region encoding the open reading frame of said protein where a genetically encoded amino acid is to be replaced by a non-genetically encoded acid.


SEQUENCE LISTING

<110> 1) Ludwig-Maximilans-Universität München 2) Bayer Schering
      Pharma AG

<120> Protein synthesis via click chemistry

<130> 46381P EP

<160> 12

<170> PatentIn version 3.3

<210> 1
<211> 258
<212> PRT
<213> Artificial

<220>
<223> YFP mut1


<220>
<221> MUTAGEN
<222> (114)..(114)
<223> Xaa = alkyne-containing lysine

<400> 1

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Phe Gly Tyr Gly Leu Gln Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Xaa Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Tyr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Arg
225                 230                 235                 240

Ser Ile Leu Lys Met Ala Ser Ala Trp Ser His Pro Gln Phe Glu Lys
                245                 250                 255

Ser Gly

<210>  2
<211>  258
<212>  PRT
<213>  Artificial

<220>
<223>  YFP mut3

<220>
<221>  MUTAGEN
<222>  (27)..(27)
<223>  Xaa = alkyne-containing lysine

<220>
<221>  MUTAGEN
<222>  (114)..(114)
<223>  Xaa = alkyne-containing lysine

<220>
<221>  MUTAGEN
<222>  (132)..(132)
<223>  Xaa = alkyne-containing lysine

<400>  2

Met Val Ser Lys Gly Glu Glu Leu Phe Thr Gly Val Val Pro Ile Leu
1               5                   10                  15

Val Glu Leu Asp Gly Asp Val Asn Gly His Xaa Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Phe Gly Tyr Gly Leu Gln Cys Phe Ala Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Xaa Phe Glu Gly Asp Thr Leu Val Asn Arg Ile Glu Leu Lys Gly
        115                 120                 125

Ile Asp Phe Xaa Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Tyr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys Arg
225                 230                 235                 240

Ser Ile Leu Lys Met Ala Ser Ala Trp Ser His Pro Gln Phe Glu Lys
                245                 250                 255

Ser Gly

<210>  3
<211>  32
<212>  DNA
<213>  Artificial

<220>
<223>  Primer

<400>  3
ggatcccatg gataaaaaac cactaaacac tc                                32

<210>  4
<211>  30
<212>  DNA
<213>  Artificial

<220>
<223>  Primer

<400>  4
gcggccgctt acaggttggt agaaatcccg                                        30


<210>  5
<211>  30
<212>  DNA
<213>  Artificial

<220>
<223>  Primer

<400>  5
gcgccgaggt gtagttcgag ggcgacaccc                                        30


<210>  6
<211>  30
<212>  DNA
<213>  Artificial

<220>
<223>  Primer

<400>  6
gggtgtcgcc ctcgaactac acctcggcgc                                        30


<210>  7
<211>  29
<212>  DNA
<213>  Artificial

<220>
<223>  Primer

<400>  7
cgtaaacggc cactagttca gcgtgtccg                                         29


<210>  8
<211>  29
<212>  DNA
<213>  Artificial

<220>
<223>  Primer

<400>  8
cggacacgct gaactagtgg ccgtctacg                                         29


<210>  9
<211>  45
<212>  DNA
<213>  Artificial

<220>
<223>  Primer

<400>  9
gcatcgactt ctaggaggac ggcaacatcc tggggcacaa gctgg                       45


<210>  10

<211>  45
<212>  DNA
<213>  Artificial

<220>
<223>  Primer

<400>  10
ccagcttgtg ccccaggatg ttgccgtcct cctagaagtc gatgc                    45


<210>  11
<211>  22
<212>  DNA
<213>  Artificial

<220>
<223>  Primer

<400>  11
catatgatgg tgagcaaggg cg                                             22


<210>  12
<211>  75
<212>  DNA
<213>  Artificial

<220>
<223>  Primer

<400>  12
ggtaccttat ccggattttt cgaattgagg atgactccat gcgctagcca tcttaaggat    60

agatctcttg tacag                                                     75